# EUROPÄISCHE PATENTSCHRIFT

(11) **EP 0 613 348 B1**
(45) Veröffentlichungstag und Bekanntmachung des Hinweises auf die Patenterteilung: **21.01.1998**
(21) Anmeldenummer: 92923288.2
(22) Anmeldetag: 10.11.1992
(51) Int. Cl.: A61B 10/00

(54) **VERFAHREN ZUR MESSUNG DER ELEKTRISCHEN LEITFÄHIGKEIT VON ZERVICAL- UND/ODER VAGINALSCHLEIM, SOWIE HIERBEI VERWANDTE MESSONDE**
PROCESS AND MEASUREMENT PROBE FOR MEASURING THE ELECTRICAL CONDUCTIVITY OF CERVICAL AND/OR VAGINAL MUCUS
PROCEDE ET SONDE DE MESURE DE LA CONDUCTIVITE ELECTRIQUE DE MUCUS CERVICAL ET/OU VAGINAL

(30) Priorität: 13.11.1991 DE 4137303
(43) Veröffentlichungstag der Anmeldung: 07.09.1994
(73) Patentinhaber: Rheintechnik Weiland & Kaspar GmbH & Co KG .Maschinenfabrik. Herstellung und Vertrieb von elektronischen, medizinischen, 56170 Bendorf/Rhein (DE)
(72) Erfinder: WEILAND, Werner, D-5413 Bendorf-Sayn (DE)
(74) Vertreter: Quermann, Helmut, Dipl.-Ing.
(86) Internationale Anmeldenummer: EP9202573
(87) Internationale Veröffentlichungsnummer: WO9309716

(56) Entgegenhaltungen:
- US-A- 4 498 481

## Beschreibung

Die Suche nach zufriedenstellenden Methoden zur Erkennung der Brunst bei landwirtschaftlichen Nutztieren ist seit langer Zeit Gegenstand wissenschaftlicher Betrachtung. Entsprechendes gilt hinsichtlich der Bestimmung des exakten Zeitpunktes des Eisprungs bei Frauen.

Um den optimalen Inseminationspunkt bei landwirtschaftlichen Nutztieren feststellen zu können, ist der Versuch unternommen worden, hormonanalytische Verfahren, Veränderungen der elektrischen Leitfähigkeit des Scheidenschleims, des vaginalen pH-Wertes, scheidenzytologische Kriterien oder die Körpertemperatur als Hilfsmittel zu nutzen. Bis heute liegen aber kaum praktisch verwendbare Methoden vor.

Aus der EP 0 177 994 ist ein Verfahren zur Voraussage des Eisprunges bei Frauen bekannt. Nach diesem wird der Anfang der Menstruation notiert und ein Angaben über den Menstruationszyklus liefernder Parameter des Körpers regelmäßig festgestellt, wobei dieser Parameter der spezifische elektrische Widerstand des Speichels des Subjektes ist. Zusätzlich wird vorgeschlagen, den elektrischen Widerstand des Schleims der Vagina beim Beginn der Ermittlung der ersten Spitze der Aufzeichnung des Widerstandswertes des Speichels festzustellen, wobei der vaginal elektrische Widerstandswert täglich festgehalten wird. Der Anstieg des vaginalen Widerstandswertes im Anschluß an einen Tiefpunkt zeigt dabei der Benutzerin das Vorliegen des Eisprunges an. In der Druckschrift ist in diesem Zusammenhang eine Meßsonde zum Ermitteln des elektrischen Widerstandes des Speichels beschrieben, diese weist eine flache Sondenoberfläche auf, in die mehrere kreisförmige Elektroden eingelassen sind. Ferner ist dort zur Ermittlung des elektrischen Widerstandes im Schleim der Vagina eine zylindrische, vorn und hinten abgerundete Meßsonde bekannt, bei der mehrere Ringelektroden vorgesehen sind. Bei beiden Meßsonden messen die Elektroden parallel.

Aufgabe der vorliegenden Erfindung ist es zunächst, ein exakt arbeitendes Verfahren zur Messung der elektrischen Leitfähigkeit von Zervical- und/oder Vaginalschleim anzugeben, ferner eine hierbei vorteilhaft Verwendung findende Meßsonde.

Die Erfindung schlägt ein Verfahren zur Messung der elektrischen Leitfähigkeit von Zervical- und/oder Vaginalschleim vor, unter Verwendung mehrerer, an unterschiedlichen Meßorten einer Körperstelle angebrachten Meßelektroden zum Zweck der Ermittlung von elektrischen Widerstandswerten, wobei die Messung mit Wechselstrom mittels einer die Meßelektroden aufweisenden Meßsonde erfolgt, wobei ferner die von den Meßelektroden jeweils ermittelten Widerstandswerte abgefragt und der geringste Widerstandswert als Bezugsgröße für die Bestimmung der elektrischen Leitfähigkeit ausgewertet wird.

Die Erfindung zieht in Betracht, daß bei der Messung der elektrischen Leitfähigkeit von Zervical- und/oder Vaginalschleim die Meßsonde und damit die Meßelektroden wegen der Bewegung von Mensch bzw. Tier ständig ihre Position zur umgebenden Körperwandung verändern, so daß die Meßelektroden mehr oder weniger den Schleim kontaktieren. Das erwünschte Meßsignal wird damit von der Menge und der räumlichen Verteilung des Schleims stark beeinflußt, das heißt es ist von der Lage des Meßortes abhängig. Dieser Abhängigkeit begegnet das erfindungsgemäße Verfahren zunächst dadurch, daß an mehreren Orten der Körperstelle gemessen wird. Signaländerungen aufgrund von Leitfähigkeitsänderungen des Schleims müssen sich damit als Tendenz bei allen Meßorten zeigen. Durch die Anordnung einer größeren Anzahl von Meßelektroden werden die verschiedenen Meßorte gewonnen. Da alle Meßelektroden einzeln geschaltet sind, besteht die Möglichkeit, zur Findung des Widerstandswertes nur diejenige Elektrode auszuwerten, die den niedrigsten Widerstandswert besitzt.

Die Auswertung des geringsten Widerstandswertes gibt Aufschluß darüber, daß diejenige Meßelektrode berücksichtigt wurde, die vom Schleim am besten umspült wurde und damit im frischesten Schleim liegt. Bezogen auf die Bestimmung des Eisprunges ist bekannt, daß der elektrische Widerstand des Zervical- und/oder Vaginalschleimes vor dem Eisprung am höchsten ist und je näher man dem Eisprung kommt geringer wird, bei gleichzeitig gegebenenfalls größer werdender Schleimmenge. Der niedrige Widerstandswert aufgrund der optimalen Plazierung der einen, für die Auswertung herangezogenen Meßelektrode und die Tendenz der Widerstandsverringerung bei einer Annäherung an den Eisprung korrelieren damit. So erhält man aufgrund des erfindungsgemäßen Verfahrens eine hohe Unabhängigkeit von Schleimmenge und Sondenlage bzw. Position der Meßelektroden, wobei man davon ausgehen kann, daß die Meßelektrode mit dem kleinsten Widerstandswert ganz von Körperflüssigkeit umspült ist. Dieser Wert wird zur Erstellung eines Algorithmuses herangezogen.

Vorteilhaft erfolgt mit den Meßelektroden eine punktuelle Messung, das heißt es finden punktförmige Elektroden Verwendung.

Die Messung der Leitfähigkeit geschieht zweckmäßig als Dauermessung in definierten Meßabständen. Es erfolgt damit eine kontinuierliche Erfassung der Leitfähigkeit des Schleims, die elektrischen Widerstandswerte werden gespeichert und können anschließend ausgewertet werden. Messungen der Meßelektroden mit anschließender Ermittlung des geringsten Widerstandswertes können beispielsweise in so kurzen Abständen wie einer viertel, halben oder einer Minute erfolgen.

Da es sich bei dem Zervical- bzw. Vaginalschleim um einen Elektrolyten handelt muß die Messung mit Wechselstrom erfolgen, da sonst Polarisationseffekte auftreten. Der ermittelte Widerstand der Körperflüssigkeit stellt sich nicht rein ohmsch, sondern als Impedanz dar. Das Meßverfahren soll so ausgelegt sein, daß sich das Ergebnis als Absolutbetrag der Impedanz ergibt.

Es wird als besonders vorteilhaft angesehen, wenn zusätzlich zu der Ermittlung der elektrischen Leitfähigkeit des Schleimes Ionenabsonderungen in dem Schleim und/oder die Körpertemperatur ermittelt werden. Die Ermittlung dieser Kontrollgrößen sollte insbesondere bei der Bestimmung des Eisprunges erfolgen.

Die Meßsonde, die zur Durchführung des Verfahrens bevorzugt Verwendung findet, weist eine größere Anzahl von Punktelektroden auf, die auf der Sondenoberfläche verteilt angeordnet sind, wobei den Punktelektroden eine gemeinsame Masse zugeordnet ist. Berücksichtigt man, daß der zwischen einem Leiterpaar, nämlich der Punktelektrode und der dazugehörigen Masse fließende Strom sich nicht auf den kürzesten Weg beschränkt, sondern sich auf jeden zur Verfügung stehenden Weg verteilt, ergeben sich zwei vorteilhafte Gestaltungen der Meßsonde. Eine erste weist konzentrisch zur jeweiligen Punktelektrode einen leitenden Massering auf, wobei zwischen dem aus der Punktelektrode und dem zugeordneten Massering gebildeten Leiterpaar ein Isolationsring angeordnet ist. Bei einer zweiten ist um die jeweilige Punktelektrode ein Isolationsring angeordnet und es weist die restliche Sondenoberfläche eine leitende Masseoberfläche auf.

Der Sondenträger für die Punktelektroden und die Masseringe bzw. die leitende Masseoberfläche bestehen selbstverständlich aus Isolationsmaterial. Um Signaländerungen aufgrund von Oxidationsvorgängen vorzubeugen, bestehen die Punktelektroden und die Masseringe bzw. Masseoberfläche aus einem nichtoxidierenden, leitfähigen Material, beispielsweise Gold.

Wird die Meßsonde in die Vagina eingeführt, ist diese zweckmäßig zylindrisch mit abgerundeten Enden oder als Kugel ausgebildet. Sie kann zusätzlich in ihre Oberfläche eingelassene ionensensitive Elektroden und/oder Temperaturelektroden aufweisen. Die Auswerteeinheiten können sich innerhalb der Meßsonde befinden, so daß sie nach der Entfernung der Meßsonde aus dem Körper in einen Speicher eingelesen werden können, es kann aber auch eine elektrische Verbindung zwischen Meßsonde und den außerhalb des Körpers angeordneten Auswerteeinheiten vorgesehen sein.

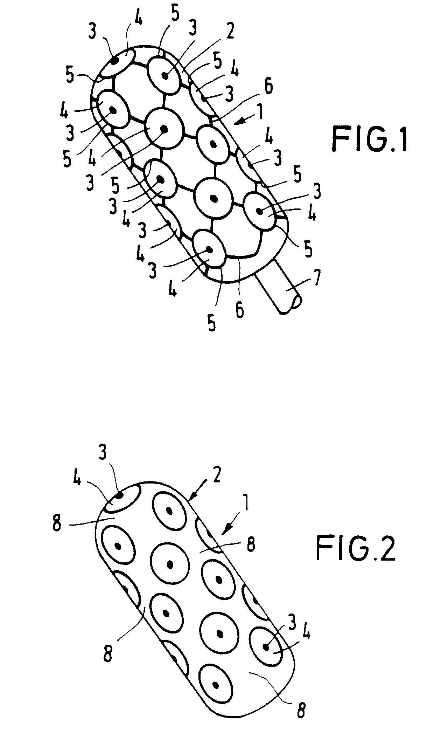

In der Darstellung der Figuren 1 bis 3 sind Details betreffend zwei mögliche Ausgestaltungen der erfindungsgemäßen Meßsonde sowie des erfindungsgemäßen Verfahrens erläutert:

Figur 1 zeigt eine Draufsicht auf eine zylindrische Meßsonde 1, deren vorderes und hinteres Ende abgerundet ist. Auf der Sondenoberfläche 2 sind eine Vielzahl von Punktelektroden 3 regelmäßig, das heißt mit gleichem Abstand zueinander, angeordnet, jede Punktelektrode 3 umgibt ein Isolationsring 4, der wiederum von einem Massering 5 umschlossen wird, der konzentrisch zur Punktelektrode 3 angeordnet. Die Punktelektroden 3 und die Masseringe 5 bestehen aus Gold. Die einzelnen Masseringe 5 sind mittels Leitungen 6 verbunden, die Punktelektroden 3 einzeln angeschlossen. Ein Anschlußkabel 7 stellt die Verbindung von in dieser Figur nicht näher gezeigten externen Aggregaten mit der Meßsonde 1 dar.

Die in Figur 2 gezeigte Meßsonde 1 ist im wesentlichen entsprechend der Meßsonde nach Figur 1 gestaltet, sie weist auf ihrer Sondenoberfläche 2 Punktelektroden 3 und diese umgebende Isolationsringe 4 auf. Statt der Masseringe 5 weist dort die zwischen den Isolationsringen 4 befindliche restliche Sondenoberfläche 8 eine leitende Masseoberfläche auf. Diese sowie die Punktelektroden bestehen aus Gold. Im Unterschied zu der Ausführungsform nach Figur 1 ist eine Auswerte- und Speichereinheit für die ermittelten Meßwerte in der Meßsonde 1 untergebracht. Die Werte werden nach dem Entfernen der Meßsonde 1 aus der Körperöffnung dargestellt.

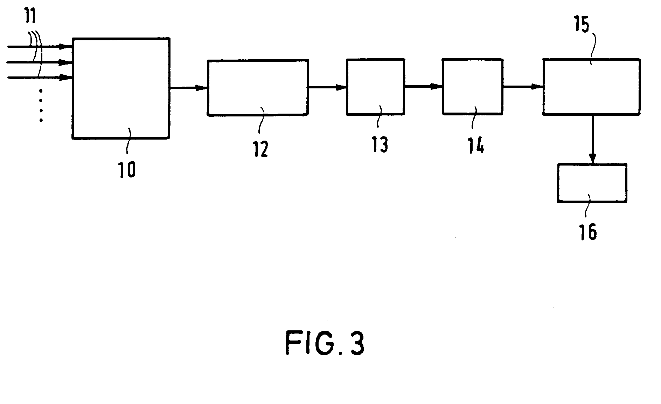

Figur 3 zeigt ein Blockschaltbild für die Brunsterkennung, beispielsweise zur Erkennung der Brunst bei Kühen. Über einen Multiplexer 10 werden die der Anzahl der Punktelektroden 3 entsprechenden Impedanzkanäle 11, beispielsweise sechzehn Impedanzkanäle an einen A/D Wandler 12 übertragen. Aus den sechzehn Werten, die zunächst in einem Zwischenpuffer 13 abgelegt werden, bestimmt eine Arithmetikeinheit (ALU) 14 den Wert mit der geringsten Impedanz. Dieser ausgewählte Impedanzwert wird in dem internen Speicher 15 abgelegt. Eine serielle Schnittstelle speist entweder eine kurze HF-Übertragungsstrecke oder eine induktive Übertragung 16. Ein externes Lesegerät empfängt den Speicherinhalt täglich und wertet die Daten zur Brunsterkennung aus. Merkmal für die Brunst ist ein deutlicher Abfall der Impedanzkurve von ca. 60 Ohm auf ca. 10 bis 20 Ohm, die grundsätzlich entsprechend der Temperaturkurve des Tieres verläuft. Eine Stromsparschaltung sollte vorgesehen sein, um die Funktionsfähigkeit der Schaltung über fünfunddreißig Tage sicherzustellen.

Die gesamte Schaltung setzt sich aus mehreren Bauteilen zusammen:

### Sensor

- Gemischt analag/digitales-ASIC:: Verstärker, Multiplexer, A/D-Wandler, Alu-Einheit (Logik Gatter), RAM
- Diskrete Technik:: Induktive Übertragung bzw. HF-Sender

### Lesegerät

- bestehend aus:: Empfangsteil (induktiv bzw. HF), µ-Prozessor, Langzeitspeicher, Ein-Ausgabeeinheiten

Sensor, ASIC und Telemetrietrecke sollten miniaturisiert aufgebaut sein, damit eine Einführung in die Vagina des Rindes möglich ist. Bei dieser Verwendung sollte die komplette Größe des Sensors auf die jeweilige Scheidenkonfiguration abgestimmt sein.

Eine entsprechende Gestaltung der Meßsonde mit kleineren Abmessungen findet zur Bestimmung des Eisprungs bei Frauen Verwendung, ferner eine entsprechende Auswerteschaltung. Insgesamt besteht die Meßsonde aus physiologisch unbedenklichem Material.

## Patentansprüche

1. Verfahren zur Messung der elektrischen Leitfähigkeit von Zervical- und/oder Vaginalschleim unter Verwendung mehrerer, an unterschiedlichen Meßorten einer Körperstelle angebrachten Meßelektroden zum Zweck der Ermittlung von elektrischen Widerstandswerten, wobei die Messung mit Wechselstrom mittels einer die Meßelektroden aufweisenden Meßsonde erfolgt, wobei ferner die von den Meßelektroden jeweils ermittelten Widerstandswerte abgefragt und der geringste Widerstandswert als Bezugsgröße für die Bestimmung der elektrischen Leitfähigkeit ausgewertet wird.

2. Verfahren nach Anspruch 1, wobei mittels der Meßelektroden eine punktuelle Messung erfolgt.

3. Verfahren nach Anspruch 1 oder 2, wobei die Messung der Leitfähigkeit als Dauermessung in definierten Meßabständen erfolgt.

4. Verfahren nach Anspruch 3, wobei die Meßabstände eine viertel, halbe oder eine Minute betragen.

5. Verfahren nach einem der Ansprüche 1 bis 4, wobei die Messung mittels der die Meßelektroden aufweisenden Meßsonde in der Zervix/Vagina zum Zwecke der Brunsterkennung bzw. Bestimmung des Eisprunges erfolgt.

6. Verfahren nach Anspruch 5, wobei sich das Ergebnis der Messung als Absolutbetrag der Impedanz darstellt.

7. Verfahren nach einem der Ansprüche 1 bis 6, wobei zusätzlich zur Leitfähigkeit des Zervical- und/oder Vaginalschleimes Ionenabsonderungen in den Schleimen und/oder die Körpertemperaturen ermittelt werden.

8. Meßsonde zur Durchführung des Verfahrens nach einem der Ansprüche 1 bis 7, wobei eine größere Anzahl von Punktelektroden (3) auf der Sondenoberfläche (2) verteilt angeordnet sind und den Punktelektroden eine gemeinsame Masse (5, 8) zugeordnet ist, derart, daß konzentrisch zur jeweiligen Punktelektrode (3) ein leitender Massering (5) angeordnet ist, sowie zwischen dem aus der Punktelektrode (3) und dem zugeordneten Massering (5) gebildeten Elektrodenpaar ein Isolationsring (4) angeordnet ist.

9. Meßsonde zur Durchführung des Verfahrens nach einem der Ansprüche 1 bis 7, wobei eine größere Anzahl von Punktelektroden (3) auf der Sondenoberfläche (2) verteilt angeordnet sind und den Punktelektroden eine gemeinsame Masse (5, 8) zugeordnet ist, derart, daß um die jeweilige Punktelektrode (3) ein Isolationsring (4) angeordnet ist und die restliche Sondenoberfläche (8) eine leitende Masseoberfläche aufweist.

10. Meßsonde nach einem der Ansprüche 8 oder 9, wobei die Punktelektroden (3) und die Masseringe (5) bzw. Masseoberfläche (8) aus nichtoxidierendem, leitfähigem Material, insbesondere Gold bestehen.

11. Meßsonde nach einem der Ansprüche 8 bis 10, wobei diese zylindrisch mit abgerundeten Enden oder als Kugel ausgebildet ist.

12. Meßsonde nach einem der Ansprüche 8 bis 11, wobei diese ionensensitive Elektroden und/oder Temperaturelektroden aufweist.

## Claims

1. Process for measuring the electrical conductivity of cervical and/or vaginal mucus, using a plurality of measurement electrodes attached at various measurement sites on a part of the body, for the purpose of ascertaining levels of electrical resistance, characterised in that the measurement process uses alternating current and a measurement probe incorporating the measurement electrodes, and that the respective resistance levels ascertained by the measurement electrodes are scanned and the lowest resistance level are evaluated as a reference variable for determining the electrical conductivity.

2. Process according to claim 1, characterised in that the measurement electrodes are used to perform point-focal measurement.

3. Process according to claim 1 or 2, characterised in that the measurement of conductivity is a long-term measurement performed at defined measurement intervals.

4. Process according to claim 3, characterised in that the measurement intervals are quarter-minute, half-minute or one-minute intervals.

5. Process according to any of claims 1 to 4, characterised in that measurement by means of the measurement probe incorporating the measurement electrodes takes place in the cervix/vagina in order to detect oestrus or determine the occurrence of ovulation.

6. Process according to claim 5, characterised in that the result of the measurement rePresents an absolute impedance value.

7. Process according to any of claims 1 to 6, characterised in that ionic secretions in mucus and/or the body temperatures are ascertained, in addition to the conductivity of the cervical and/or vaginal mucus.

8. Measurement Probe for carrying out the process according to any of claims 1 to 7, characterised in that a fairly large number of point electrodes (3) are distributed over the probe surface (2) and a common earth (5, 8) is assigned to the point electrodes, in such a manner that a conducting ring earth (5) is arranged concentrically to the respective Point electrode (3), and an insulation ring (4) is arranged between the electrode Pair formed by the point electrode (3) and the ring earth (5) assigned to it.

9. Measurement probe for carrying out the process according to any of claims 1 to 7, characterised in that a fairly large number of Point electrodes (3) are distributed over the probe surface (2) and a common earth (5, 8) is assigned to the point electrodes, in such a manner that an insulation ring (4) is arranged around the respective Point electrode (3) and the remaining probe surface (8) has a conducting earth surface.

10. Measurement probe according to either of claims 8 or 9, characterised in that the point electrodes (3) and the earth rings (5) and earth surface (8) consist of non-oxidising, conductive material, in particular gold.

11. Measurement probe according to any of claims 8 to 10, characterised in that it is a cylindrical construction with rounded ends or else a sphere.

12. Measurement probe according to any of claims 8 to 11, characterised in that it has ion-sensitive electrodes and/or temperature electrodes.

## Revendications

1. Procédé de mesure de la conductivité électrique du mucus cervical et/ou vaginal en utilisant plusieurs électrodes de mesure appliquées en différents points de mesure d'une zone corporelle dans le but de détecter des valeurs de la résistance électrique,
procédé dans lequel
la mesure a lieu avec du courant alternatif au moyen d'une sonde de mesure comportant les électrodes de mesure, dans lequel en outre on lit les valeurs de la résistance respectivement détectées par les électrodes de mesure et on exploite la plus petite valeur de la résistance comme grandeur de référence pour la détermination de la conductivité électrique.

2. Procédé selon la revendication 1,
dans lequel
une mesure ponctuelle a lieu au moyen des électrodes de mesure.

3. Procédé selon la revendication 1 ou 2,
dans lequel
la mesure de la conductivité électrique a lieu comme mesure permanente dans des intervalles de mesure définis.

4. Procédé selon la revendication 3,
dans lequel
les intervalles de mesure se montent à un quart, une demiminute ou une minute.

5. Procédé selon une des revendications 1 à 4,
dans lequel
la mesure au moyen de la sonde de mesure comportant les électrodes de mesure a lieu dans le cerveau ou le vagin dans le but de reconnaître la période de saillie (animaux) ou de déterminer la période d'accouplement (humains).

6. Procédé selon la revendication 5,
dans lequel
le résultat de la mesure se présente comme la valeur absolue de l'impédance.

7. Procédé selon une des revendications 1 à 6,
dans lequel
additionnellement à la conductivité du mucus cervical et/ou vaginal, on détermine des sécrétions ioniques dans les mucus et/ou les températures du corps.

8. Sonde de mesure pour la réalisation du procédé selon une des revendications 1 à 7,
dans laquelle
un assez grand nombre d'électrodes électriques ponctuelles (3) est monté réparti sur la surface de la sonde (2) et une masse commune (5, 8) est associée aux électrodes ponctuelles, de manière que, concentriquement à l'électrode ponctuelle respective (3), est disposé un anneau de masse conducteur (5) ainsi qu'entre la paire d'électrodes formée de l'électrode ponctuelle (3) et de l'anneau de masse associé (5) est disposé un anneau isolant (4).

9. Sonde de mesure pour mettre en oeuvre le procédé selon une des revendications 1 à 7,
dans laquelle
un assez grand nombre d'électrodes ponctuelles (3) est disposé réparti sur la surface de la sonde (2) et une masse commune (5, 8) est associée aux électrodes ponctuelles de manière que, autour de chaque électrode ponctuelle (3), est placé un anneau isolant (4) et la surface de la sonde restante (8) comporte une surface de masse conductrice.

10. Sonde de mesure selon une des revendications 8 ou 9,
dans laquelle
les électrodes ponctuelles (3) et les anneaux de masse (5) ou surface de masse (8) sont composés d'un matériau conducteur, non oxydant, en particulier l'or.

11. Sonde de mesure selon une des revendications 8 à 10,
dans laquelle
celle-ci est réalisée de manière cylindrique avec des extrémités arrondies ou en tant que bille.

12. Sonde de mesure selon une des revendications 8 à 11,
dans laquelle
celle-ci comporte des électrodes sensitives aux ions et/ou des électrodes de température.
